# EUROPEAN PATENT APPLICATION

(11) **EP 1 974 740 A1**
(43) Date of publication of application: **01.10.2008**
(21) Application number: 05807908.8
(22) Date of filing: 24.10.2005
(51) Int. Cl.: A61K 38/18, A61K 38/08, A61K 38/10, A61P 37/02

(54) **USE OF TGF-BETA 1 INHIBITOR PEPTIDES IN THE PREPARATION OF AN IMMUNE RESPONSE MODULATING AGENT**

(71) Applicant: Proyecto de Biomedicina Cima, S.L., 31180 Cizur Mayor (Navarra) (ES)
(72) Inventor: BORRÁS CUESTA, Francisco, E-31008 Pamplona (ES); CASARES AGAR, Noelia, E-31008 Pamplona (ES); DOTOR DE LAS HERRERÍAS, Javier, E-31008 Pamplona (ES); GIL GUERRERO, Lucia, E-31008 Pamplona (ES); LASARTE SAGASTIBELZA, Juan José, E-31008 Pamplona (ES); SAROBE UGARRIZA, Pablo, E-31008 Pamplona (ES); PRIETO VALTUEÑA, Jesus, E-31008 Pamplona (ES)
(74) Representative: ABG Patentes, S.L.
(86) International application number: PCT/ES2005/000569
(87) International publication number: WO 2007/048857

(57) **Abstract**

The present invention relates to the use of a peptide inhibiting TGF-β1 selected from: peptide p144 whose sequence corresponds to SEQ ID NO: 1, peptide p17 whose sequence corresponds to SEQ ID NO: 2, a peptide which has at least 90% homology therewith, or fragments of the above, in the preparation of an immune response modulating agent.

SEQUENCE LISTING

<110> PROYECTO DE BIOMEDICINA CIMA S.L.

<120> USE OF TGF-β1 INHIBITOR PEPTIDES IN THE PREPARATION OF AN
IMMUNE RESPONSE MODULATING AGENT

<130> 5.050.034/ZAR

<160> 5

<170> PatentIn version 3.1

<210> SEQ ID NO: 1
<211> 14
<212> Peptide
<213> Synthetic peptide from human TGBβ1 type III receptor
<220> Domain: 730-743

<400> 1
Thr Ser Leu Asp Ala Ser Ile Ile Trp Ala Met Met Gln Asn
 1               5                   10

<210> SEQ ID NO: 2
<211> 15
<212> PRT
<213> Artificial peptide sequence

<400> 2
Lys Arg Ile Trp Phe Ile Pro Arg Ser Ser Trp Tyr Glu Arg Ala
 1               5                   10                  15

<210> SEQ ID NO: 3
<211> 11
<212> PRT
<213> Artificial peptide sequence

<400> 3
Lys Arg Ile Trp Phe Ile Pro Arg Ser Ser Trp
 1               5                   10

<210> SEQ ID NO: 4
<211> 11
<212> PRT
<213> Artificial peptide sequence

<220>
<221> PEPTIDE
<222> (11)...(111)

<223> amidated

<400> 4
Lys Arg Ile Trp Phe Ile Pro Arg Ser Ser Trp
 1               5                   10


<210> SEQ ID NO: 5
<211> 11
<212> PRT
<213> Artificial peptide sequence

<220>
<221> PEPTIDE
<222> (1)...(1)
<223> acetylated

<220>
<221> PEPTIDE
<222> (11)...(11)
<223> amidated

<400> 5
Lys Arg Ile Trp Phe Ile Pro Arg Ser Ser Trp
 1               5                   10

## Description

### TECHNICAL FIELD OF THE INVENTION

The present invention is included in the field of the preparation of immune response modulating agents.

### STATE OF THE ART

TGF-β1 (transforming growth factor β1) is a potent immunomodulator that is present in all phases of the immune response generating different effects. It is currently known as a potent immune system cell regulator, including lymphocytes, macrophages and dendritic cells (Letterio J.J., 1998).

The biological activity of TGF-β1 varies a great deal depending on the type and state of cell differentiation, as well as the presence of other cytokines, suggesting that an alteration in the balance of this set of cytokines can also affect TGF-β1 and contribute to the development of pathologies associated with the dysfunction of the immune system. TGF-β1 regulates the immune response in a complex, context-dependent manner, which has been revealed by using experimental models of different diseases, as well as for the evaluation of genetically modified mice with regard to TGF-β1 expression, its receptors or regulating proteins. TGF-β1 regulates the function and interaction of immune system cells in the development of humoral, cytotoxic and immunotolerance responses and the pathological origin of many infectious and autoimmune diseases.

T lymphocytes are clearly regulated by TGF-β1 in all phases of their development (Fontana A. et al., 1992). The effect of TGF-β1 varies according to the state of differentiation of the lymphocyte and the type of activation signal that it has received. The first studies on the effect of TGF-β1 in human lymphocytes revealed their capacity to produce and secrete TGF-β1 as inhibitor of IL-2 dependent proliferation and cytolytic function (Pardoux C. et al., 1997).

Dendritic cells are a leukocyte population which is clearly differentiated due to their function as antigen presenting cells in the activation of T lymphocyte responses. They are a highly specialised cell population, which include epidermal Langerhans cells and follicular dendritic cells from the lymph nodes, and wherein TGF-β1 regulates both their differentiation and their activity (Strobl H, Knapp W., 1999).

It has been identified that TGF-β1 boosts the *in vitro* functional differentiation of dendritic cells, from CD34⁺ precursors, induced by the presence of other cytokines (TNF-α, SCF and GM-CSF). TGF-β1 also acts by increasing the viability of the dendritic cells in culture. On the other hand, the role of TGF-β1 in this cell type also seems to be related to a regulation mechanism which inhibits low specificity responses to avoid autoimmune processes.

In the differentiation, proliferation and production of B cell Ig (immunoglobins), TGF-β1 has a regulating role via the inhibition of the levels of certain surface molecules, including the major histocompatibility complex type II (MCH-II) both in pre-B lymphocytes and mature B cells. On the other hand, TGF-β1 inhibits Ig secretion in general, but clearly induces the production of IgA for which reason it performs an important role in the immune response associated to mucous membranes. Most studies on the effect of TGF-β1 as inhibitor of the production of all types of Ig, have been performed *in vitro.* However, the need for certain levels of TGF-β1 in lymphocyte culture, which act at an autocrine level, for the effective production of IgG and IgE has also been described. Thus, the function of TGF-β1 in the induction of antibodies is, as in many other processes, dual and opposing according to the context of the immune response (Lebman D.A., Edmiston J.S., 1999).

In the case of macrophages, the effect of TGF-β1 at a tissue level is generally suppressant and contributes to finalizing the inflammatory response.

Possibly, the most relevant effect of TGF-β1 on the inactivation of macrophages is due to its capacity for limiting the production of oxygen reactive species and metabolic intermediaries of nitrogen by cells activated with IFN-γ or LPS. The enzyme responsible for NO (nitric oxide) production by the activated macrophages is the inducible form of the nitric oxide synthase (iNOS) enzyme. The regulation of the activity of this enzyme by different cytokines, including TGF-β1, permits the regulation of the immune response in general, and in particular, the response of the macrophages to microorganisms and tumour cells. TGF-β1 inhibits the iNOS enzyme both at a transcriptional level, reducing mRNA levels, and suppressing protein activity. TGF-β1 also inhibits the production of intermediary oxygen reactive species and oxidative cytotoxicity, by the inactivation of macrophages and control of peripheral blood monocytes (Ashcroft G.S., 1999).

Additionally, the activation or production of TGF-β1, as well as the alternation of its signalling pathway, is described in many diseases produced by the infection of different microorganisms, including *Leishmania, Trypanosoma cruzi,* human immunodeficiency virus, hepatitis C virus, ...

The documents in the state of the art closest to the present invention are the patent ES 2 146 552 and patent application ES200302020. The first document relates to the use of antagonist peptides of the TGF-β1 bond to its receptors in the organism, characterized in that it has partial sequences of amino acids which are similar or identical to those of TGF-β1 and/or its receptors; as well as its use to prepare a composition of application in liver diseases, in particular for hepatic fibrosis. This document protects peptide p144 (SEQ ID NO: 1) as well as its aforementioned use, although it does not mention its use in the preparation of an immune response modulating agent which constitutes the object of the present invention.

Patent application ES200302020 protects peptides inhibiting the biological activity of TGF-β1 which have been produced from a library of phages, and their use for the treatment of diseases which progress with a deregulated expression of TGF-β1, particularly fibrotic alterations. This document protects peptide p17 (SEQ ID NO: 2) and the use disclosed, but again it does not relate to its use in the preparation of an immunomodulating agent.

This immune system modulating effect is very important as it permits stimulating or inhibiting different aspect of the immune response according to requirements, and it may even have applications as a vaccination adjuvant.

Another relevant document is the patent application WO 2005/059133A2, which relates to a pharmaceutical composition which comprises at least one stimulator of immune cell function and at least one substance that inhibits cell proliferation and/or induces cell death. An antagonist of TGF-β1 is used as stimulator of the immune system function, selected from: oligonucleotides which hybridise with mRNA or with the DNA that encodes TGF-β1, TGF-β1-inhibiting proteins and peptides which have a molecular weight lower than 100 kDa which inhibit TGF-β1. Additionally, this document relates to the use of said pharmaceutical composition in the treatment of neoplasms. Nevertheless, this document does not use any peptide as TGF-β1 inhibitor, but instead an oligonucleotide, although it can be deduced that a peptide of these characteristics would have, in principle, a similar effect. This cannot be affirmed without considerable experimentation.

Below, a list of the bibliography cited in the present application is shown:
Ashcroft GS. (1999). Bidirectional regulation of macrophage function by TGF-beta. Microbes Infect. Dec;1(15) : 1275-82.
Fontana A, Constam DB, Frei K, Malipiero U, Pfister HW. Modulation of the immune response by transforming growth factor beta. (1992) Int Arch Allergy Immunol.; 99(1):1-7.
Lai, M.Z., Ross, D.T., Guillet, J.G., Briner, T.G., Getter, M.L., Smith, J.A. (1987). T lymphocyte response to bacteriophage lambda repressor cI protein. Recognition of the same peptide present by Ia molecules of different haplotypes. J. Immunol 139, 3973-80.
Letterio, J.J., Roberts, A.B. (1998). Regulation of immune responses by TGF-beta. Annu Rev Immunol 16, 137-761.
Lebman DA, Edmiston JS. (1999). The role of TGF-beta in growth, differentiation and maturation of B lymphocytes. Microbes Infect. Dec;1 (15) : 1297-304.
Pardoux, C., Ma, X., Gobert, S., Pellegrini, S., Mayeux, P., Gay, F., Trinchieri, G., Chouaib, S. (1999). Downregulation of interleukin-12 (IL-12) responsiveness in human T cells by transforming growth factor-beta: relationship with IL-12 signaling. Blood 93, 1448-55.
Schini, V.B., Durante, W., Elizondo, E., Scott-Burden, T., Junquero, D.C., Schafer, A.L, Vanhouette, P.M. (1992). The induction of nitric oxide synthase activity is prohibited by TGF-beta 1, PDGFAB and PDGFBB in vascular smooth muscle cells. Eur J Pharmacol 216, 379-83.
Strobl H, Knapp W. (1999). TGF-betal regulation of dendritic cells. Microbes infect. Dec; 1(15) :1283-90.
Teicher B.A. (2001). Malignant cells, directors of the malignant process: Role of transforming: Role of transforming growth factor-beta. Cancer and Metastasis Reviews 20, 133-143.

### DESCRIPTION OF THE INVENTION

In order to facilitate understanding of the present text it is indicated that the term "peptide p144" refers to a peptide inhibiting TGF-β1 characterized in that its sequence of amino acids corresponds to that defined in SEQ ID NO:1. Likewise, the term "peptide p17" refers to a peptide inhibiting TGF-β1 activity, characterized in that its sequence of amino acids corresponds to that defined in SEQ ID NO: 2.

"Freund's incomplete adjuvant" refers to a composition that is very well known by a person skilled in the art, characterized in that it is composed of a water-in-oil emulsion, which acts as adjuvant delaying antigen release.

The present invention relates to an immune response modulating agent characterized in that it comprises a peptide inhibiting TGF-β1 selected from: peptide p144 whose sequence corresponds to SEQ ID NO: 1, peptide p17 whose sequence corresponds to SEQ ID NO: 2, a peptide which has at least 90% homology therewith, or fragments of the above. In a particular embodiment of the invention, said fragment of a peptide inhibiting TGF-β1 is selected from: fragment p17(1-11) defined in the sequence SEQ ID NO: 3, fragment p17(1-11)am which corresponds to SEQ ID NO: 4, and fragment Acp17(1-11)am defined by sequence SEQ ID NO:5.

On the other hand, the invention also relates to the use of said modulating agent in the regulation of humoral or cellular immune responses, or both. In a preferred embodiment, the invention relates to the use of the modulating agent as vaccination adjuvant. In a particular embodiment of the invention, the immune response modulating agent is characterized in that it further comprises Freund's incomplete adj uvant.

In a preferred embodiment, the invention relates to the use of said modulating agent in the preparation of a pharmaceutical composition for the treatment of pathologies selected from: pathologies related to microorganisms which induce an immunosuppression mediated by TGF-β1 and cancer. Preferably, said microorganisms are selected from *Leishmania, Trypanosoma cruzi,* human immunodeficiency virus, the flu virus, and the herpes simple virus. Likewise, in a particular embodiment of the invention, the aforementioned composition is for the treatment of a cancer selected from: breast cancer, prostate cancer, colon carcinoma, pancreatic cancer, skin cancer, hepatocarcinoma, multiple myeloma and stomach cancer.

The present invention relates to the use of a peptide inhibiting of TGF-β1 selected from: peptide p144 whose sequence corresponds to SEQ ID NO: 1, peptide p17 whose sequence corresponds to SEQ ID NO: 2, a peptide which has at least 90% homology therewith, or fragments of the above, in the preparation of an immune response modulating agent.

Furthermore, the invention relates to a method for the use of a peptide inhibiting TGF-β1 selected from: peptide p144 whose sequence corresponds to SEQ ID NO: 1, peptide p17 whose sequence corresponds to SEQ ID NO: 2, a peptide which has at least 90% homology therewith, or fragments of the above, to prepare an immune response modulating agent.

Additionally, the invention relates to the use of a fragment of a peptide inhibiting TGF-β1 obtained from peptide p17 selected from: fragment p17(1-11) defined in the sequence SEQ ID NO: 3, fragment p17(1-11)am which corresponds to SEQ ID NO: 4, and fragment Acp17(1-11)am defined by sequence SEQ ID NO:5. To facilitate understanding of the text, it is indicated that peptide p17(1-11)am corresponds to a fragment which corresponds to amino acids 1 to 11 of peptide p17, wherein the amino acid in position 11 (tryptophan) is amidated; Acp17(1-11)am corresponds to the previous fragment which further has the amino acid in position 1 (lysine) acetylated.

The present invention also relates to peptides which have at least 70% homology with said peptides, and preferably that have at least 80% homology with them, provided that they maintain the capacity to inhibit the biological activity of TGF-β1. As well as any fragment of the above which maintains the capacity of inhibiting the biological activity of TGF-β1.

In a preferred embodiment, the present invention relates to the use of an aforementioned peptide inhibiting TGF-β1, characterized in that the aforementioned modulating agent regulates the humoral or cellular immune responses, or both. In a particular embodiment of the present invention, said modulating agent has a stimulating effect on the immune response, preferably as vaccination adjuvant.

On the other hand, a preferred embodiment of the present invention is characterized in that said modulating agent has an inhibiting effect on immune response.

Additionally, the invention relates to the use of a DNA sequence that encodes a peptide inhibiting TGF-β1 selected from: peptide p144 whose sequence corresponds to SEQ ID NO: 1, peptide p17 whose sequence corresponds to SEQ ID NO: 2, a peptide which has at least 90% homology therewith, or fragments of the above, to prepare an immune response modulating agent. The invention further relates to the use of a recombinant expression system that encodes peptide p144, peptide p17, a peptide which has at least 90% homology therewith, or fragments of the above, to prepare an immune response modulating agent. In a preferred embodiment of the invention, said immune response modulating agent has an effect selected from: immune response stimulator and inhibitor.

On the other hand, the invention relates to the use of a peptide inhibiting TGF-β1 whose sequence corresponds to SEQ ID NO: 1, a peptide which has at least 90% homology therewith, or fragments of one of the above in the preparation of a composition for the treatment of pathologies selected from: pathologies related to microorganisms which induce an immunosuppression mediated by TGF-β1 and cancer. In a particular embodiment of the present invention, said microorganisms are selected from: *Leishmania, Trypanosoma cruzi,* human immunodeficiency virus, the flu virus, and the herpes simple virus.

A particular embodiment of the present invention is characterized in that said composition would have an effect on the induction of immune responses to established tumours, inhibiting the immunosuppressant effect associated to the production and/or activation of TGF-β1 in various types of tumours (Teicher B.A., 2001): breast cancer, prostate cancer, colon carcinoma, pancreatic cancer, skin cancer, hepatocarcinoma, multiple myeloma and stomach cancer.

The modulating agent object of the present invention can be used in all kind of mammals, including rodents and primates. And, in a preferred embodiment, in human beings.

### GRIEF DESCRIPTION OF THE FIGURE

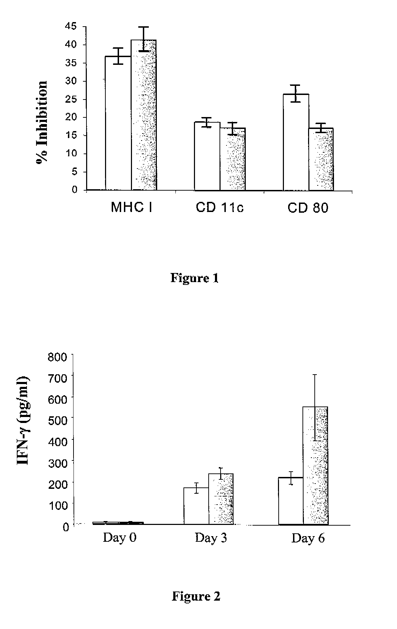

- **Figure 1.**: Inhibition of different markers expression during dendritic cells maturation after incubation with p144 (full bars) or with antibodies neutralizing TGF-β1 (empty bars). The cell surface markers expression was measured by flow cytometry.
- **Figure 2.**: Effect of the administration of p144 and/or RAd-IL12 to BALB/c mice on the serum levels of IFN-γ on days 0, 3 and 6. 10⁸ pfu of mouse RAd-IL12 (empty bars) or RAdIL-12 and p144 (full bars) were administered intraperitoneally.

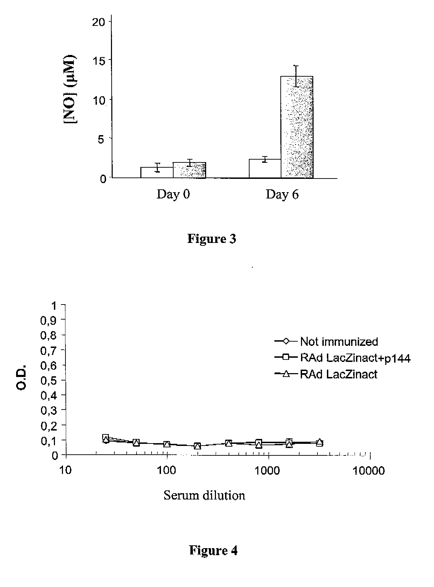

- **Figure 3.**: NO levels (µM) in serum on days 0 and 6 after the administration of 10⁸ pfu of mouse RAd-IL12, by intraperitoneal route together with p144 (full bars) or without p144 (empty bars).
- **Figure 4.**: Humoral response to RAd LacZ induced on day 15 in BALB/c mice after a subcutaneous immunization (in FIA) with RAd LacZinact in the presence or absence of p144.

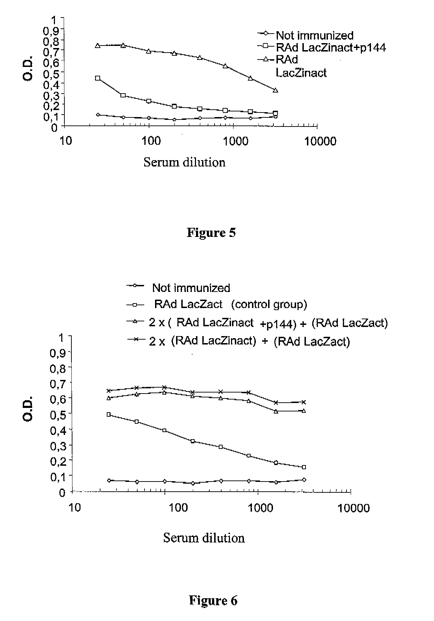

- **Figure 5.**: Humoral response to RAd LacZ induced on day 15 in BALB/c mice after a second subcutaneous immunization (in FIA) with RAd LacZinact in the presence or absence of p 144.
- **Figure 6.**: Humoral response induced in the mice of Figure 5 on the 7^{th} day after the infection by intravenous route with 4 x 10⁸ pfu of RAd LacZact. The control group corresponds to mice administered just once intravenously with 4x10⁸ pfu of RAd LacZact.

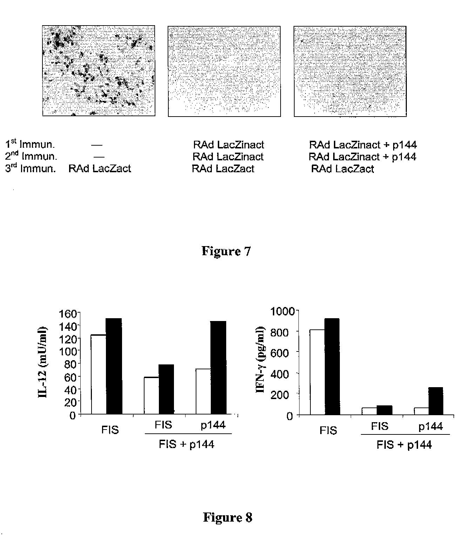

- **Figure 7.**: X-gal stains of histological cuts of liver samples from the mice of Figure 6, 7 days after the intravenous administration of 4x10⁸ pfu of RAd LacZact.
- **Figure 8.**: Effect of p144 inclusion in the immunization mixtures with FIS, on the levels of IL-2 (A) and IFN-γ (B) in supernatants of lymphocyte cultures, derived from nodes obtained from mice immunized only with FIS or with FIS+p144. The production of cytokines was measured *in vitro* after restimulation of the cultures with 6 µM (empty bars) or 30 µM (full bars) of FIS or p144.

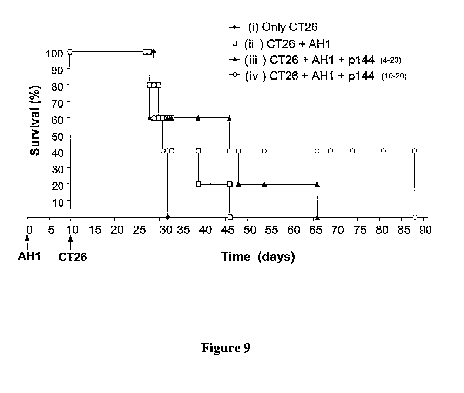

- **Figure 9.**: Survival of BALB/c mice which were administered 5x10⁵ CT26 cells by intravenous route and which received different treatments (i) - (iv). With the exception of the control group which only received 5x10⁵ CT26 cells on day 10, the three remaining groups were further immunized with 50 µg of AH1 in FIA by subcutaneous route on day 0. Groups (iii) and (iv) further received 50 µg of p144 by intraperitoneal route on alternate days between days 4-20 and 10-20, respectively.

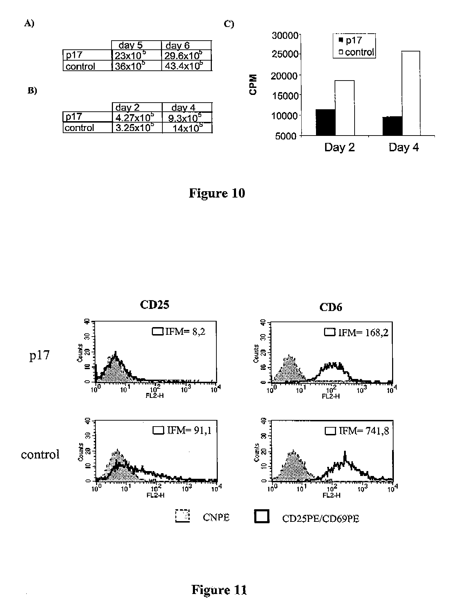

- **Figure 10.**: The addition of the TGF-β1 blocking peptide p17 to the mouse NK cell culture medium inhibits their proliferation in response to high IL-2 concentrations. In all cases, "Natural Killer" lymphocytes established exactly in the same way with and without peptide p17 were compared. **A**) Cell count at the times indicated from a culture of total splenocytes of RAG^{-/-} mice. The value shown corresponds to the mean of the counts of 2 x 3.5 cm diameter wells, in each case in absolute number. **B**) Count at the times indicated of a DX5+ cell culture, purified magnetically from splenocytes of Rags^{-/-} mice, which lack T and B lymphocytes, represented as mean of the counts of 2 x 0.4 cm diameter wells in each case. **C**) Proliferation, of microculture cells similar to those represented in B and at those same times, measured as incorporation of tritium labelled thymidine in a 6 hour assay.
- **Figure 11.**: Peptide p17 reduces the expression levels in membrane of the activation markers CD25 and CD69 in mouse NK cells. The histograms show the expression level by flow cytometry of these markers in cells cultured with and without peptide and activated with IL-2. The mean fluorescence identity (MFI) of each marker is indicated inside each histogram.

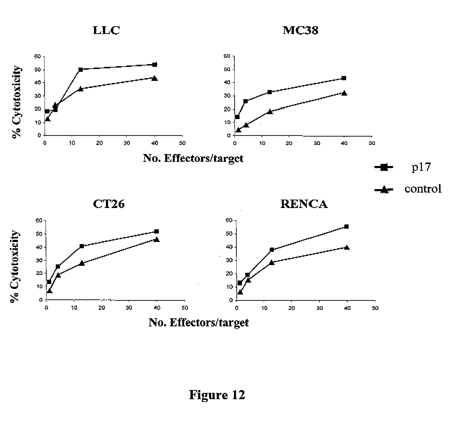

- **Figure 12.**: Peptide p17 increases the cytotoxicity of mouse NK cells, activated with IL-2 against various tumour lines. The graphics show the lysis percentages of cell lines with different sensitivity to cytotoxicity by NK. The effector cells were maintained with or without peptide for 6 days in culture and during the chromium release assay time on the target cells indicated at the corresponding proportions between effector lymphocytes and target cells.

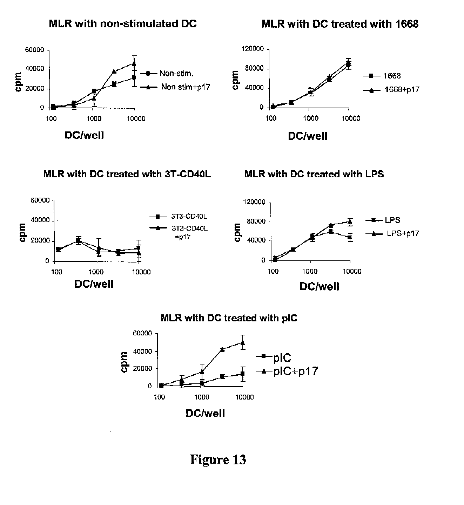

- **Figure 13.**: The graphics show the measured cell proliferation, as an incorporation of tritium labelled thymidine, in accordance with the quantity of dendritic cells present per well, in the absence or presence of different prior stimuli and in the presence or absence of peptide p17 (150 gg/ml). Peptide p17 increases the lymphocyte proliferation in mixed leukocyte response (MLR) assays with CD non-stimulated or stimulated with LPS or pIC.

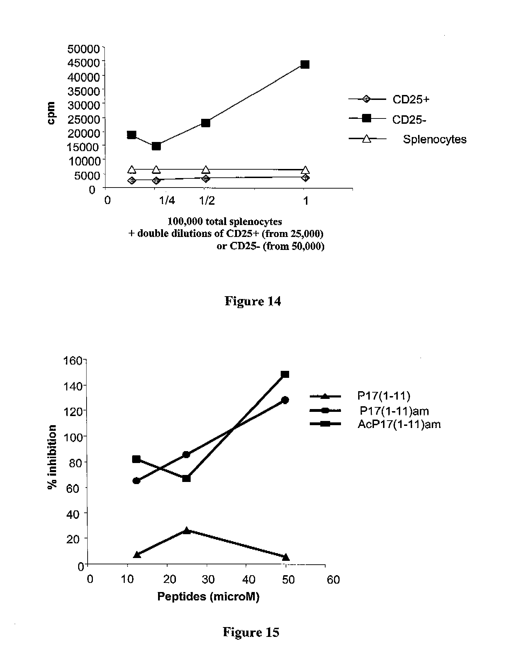

- **Figure 14.**: The CD25 population generates a suppressant effect on the proliferation of splenocytes activated by Anti-CD3 antibodies ( 0.5 µl/well) (rhombus), the CD25- (square) cells are incapable of generating this effect, permitting cell proliferation compared with the basal proliferation of splenocytes in the absence of proliferative stimuli (triangles).
- **Figure 15.**: The peptides inhibiting TGF-β (truncated and modified from p17) in a coculture of regulatory T cells and activated mouse splenocytes inhibit the suppressant action of cell proliferation exerted by the regulatory T lymphocytes. The dose-dependent effect of the peptides p17(1-11)am and Acp17(1-11)am on the inhibitory action of regulatory T lymphocytes can be observed. The inhibition exerted is dose-dependent, at a concentration of 50 µM, p17(1-11) is capable of inhibiting the suppressant effect by 20%, p17(1-11)am by 128% and Acp17(1-11)am by 148% at a concentration of 25 µM.

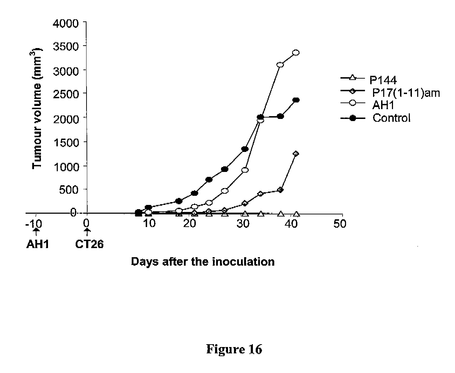

- **Figure 16.**: The peptides inhibiting TGF-β, p144 and p17(1-11)am administered by intraperitoneal route between days 6 and 10 (50 µg/mouse/48h) delay tumour growth from the subcutaneous inoculation of 50,000 CT26 cells on day 0, in animals immunized 10 days previously with AH1.

### EMBODIMENT OF THE INVENTION

Below, some examples of functioning are shown of the invention with illustrative character, and in a way which by no means limits the scope thereof, are shown.

### EXAMPLE 1

This example studies the effect of peptide p144 in a system wherein exogenous TGF-β1 is used as inducer of the differentiation of a population of splenocytes towards dendritic cells.

### Isolation and culture of dendritic cells

After sacrificing an 8-week old male C57 mouse, its spleen was extracted in sterile conditions and it was homogenised on a plate with clean medium to produce a cell suspension. The cells were centrifuged for 5 minutes at 1000 rpm and the cell sediment obtained was resuspended with 1 ml/spleen of ACK lysis solution (0.15M NH₄Cl, 1mM KHCO₃, 0.1M sodium salt-EDTA solution, pH 7.2-7.4) for 1 minute at 37°C. Next, the cells were centrifuged and washed with 10 ml of cold R10 medium (RPMI-1640, 10% FBS, Glutamine, 2 x 10⁻⁵ M 2-Mercaptoethanol) to centrifuge and wash once more. Finally, the cells were resuspended in 50 ml of R10 medium. 6-well plates were prepared (Costar #3471) treating them with 1 ml per well of R10 medium to differentiate splenocytes from dendritic cells [R10 + 10 ng/ml of mouse GM-CSF (Peprotec, EC LTD, London, UK) + 1 ng/ml of TGF-β1 (RD Systems, Minneapolis, USA)], for 15 minutes at room temperature. Next, 2 ml of the cell suspension were added to each well and they were incubated at 37°C and 5% CO₂ During the first weeks, the medium was changed twice, by the elimination of 1 ml of supernatant and the addition of 1 ml of fresh medium (R10 + GM-CSF + TGF-β1) for dendritic cells. After these two weeks, the medium was separated. 1 ml per well of dissociation medium in enzyme-free BPS (GIBCO BRL) was added to the plates, and the medium was incubated at 37°C, then it was removed and 2 ml of tepid medium (R10 + GM-CSF + TGF-β1) was added. Next, the cells were lifted with slow pipetting and the lifted cells were centrifuged at 1000 rpm and they were resuspended in fresh medium (R10 + GM-CSF + TGF-β1). The cells thus obtained can be reamplified by seeding on a plate, repeating the initial process.

### Treatment of dendritic cells

The following treatments were performed during 72 hours on an 18-day culture of dendritic cells derived from splenocytes.
- Control group: cells treated with medium for dendritic cells (GM-CSF + TGF-β1), with 0.25% DMSO.
- Anti (TGF-β1) antibody: cells treated with medium for dendritic cells (GM-CSF + TGF-β1), with 0.25% DMSO, to which an anti (TGF-β) neutralizing antibody (Pharmingen) was added at a concentration of 20 µg/ml.
- Peptide p144: cells treated with medium for dendritic cells (GM-CSF + TGF-β1), to which peptide p144 was added in solution with DMSO, there remaining a final peptide solution of 50 µg/ml at 25% DMSO.

After 48 hours, the media corresponding to each treatment were renewed and at the end of the treatment, the cells were collected by pipetting, after treatment with dissociation media (GIBCO BRL).

### Analysis of surface markers by flow cytometry

The determination of surface markers, from the splenocytes cultured with the different treatments, was performed using flow cytometry (FACScalibur, Becton-Dickinson, San Jose, CA, USA). The cells were washed with 2 ml of saline solution per well and then 1 ml of dissociation medium in enzyme-free PBS (GIBCO BRL) was added, and it was incubated for 10 minutes at 37°C. The medium was then removed and 2 ml of PBS were added. The cells were lifted by slow pipetting and the lifted cells were centrifuged at 1000 rpm at a concentration of 2 x 10⁶ cell/ml in PBS. 100 µl/well of the cell suspension obtained were incubated, with 1 µl of the phials of mouse anti-CD80, anti-CD11c and anti-MHC I monoclonal antibodies (Becton-Dickinson, Pharmingen), conjugated with FITC (1 mg/ml), in 96-well plates for 30 minutes at 4°C and in the dark. Then, the cells were washed 3 times with PBS by centrifuging the plate at 1500 rpm (Centrifuge 5810R, eppendorf) for 5 minutes, eliminating the supernatant and resuspending the cells in 100 µl of PBS. As a negative control, a non-reactive monoclonal antibody was used, conjugated with FITC (Becton-Dickinson, Pharmingen).

During the maturation of splenocytes *in vitro,* the presence of certain factors and cytokines in the medium may differentiate the cells towards different leukocyte phenotypes. In this case, TGF-β1 is described as a necessary factor *in vitro* so that certain cell types express markers associated to the dendritic cells on the surface. When we study the effect of the incubation of dendritic cells during 72 hours in the presence of p144 on the markers of these cells, it was observed that p144 had a negative effect on MHC I, CD11c and CD80 expression. This effect will have the same sense and similar magnitude as that produced when the cells are incubated in the presence of anti-(TGF-β1) antibodies. Indeed, as can be observed in figure 1, the fluorescence levels associated with each marker are reduced in a similar manner by treatment with p144 or anti-(TGF-β1) antibody. This result reveals the importance of TGF-β1 in the maturation of the dendritic cells and suggests that the use of inhibitors of this cytokine in immunization protocols could have important effects on both the induction of humoral and cell responses.

### EXAMPLE 2

### In vivo activity of a recombinant adenovirus for mouse IL-12

This example studies the effect of peptide p144 in an *in vivo* system wherein TGF-β1 acts as supposed antagonist of the cytokines induced in this model. The production of mouse IL-12 by the expression of a transgen included in the recombinant adenovirus, induces an inflammatory state through the induction of a cascade of factors, including IFN-γ and nitric oxide. TGF-β1 has been described as an inhibitor of the production and biological action of IL-12, IFN-γ and NO (Pardoux C. et al., 1999; Schini V. B. et al., 1992). In this model, 1x10⁸ pfu of mouse RAd IL-12 were intraperitoneally administered in 500 µl of saline serum to groups of 3 BALB/c mice, from 4 to 8 weeks old (Harlan), distributing the animals in the following groups:
- **Rad IL-12:** these animals received 1x10⁸ pfu of mouse RAd IL-12 on day 0.
- **Rad IL-12** + **p144:** these animals received the same treatment as the previous group but they were administered peptide for 5 days, after the administration of the adenovirus, at a daily dose of 100 µg of PS which contained 0.66% DMSO.

Blood samples were extracted from both groups on days 6 and 9 after immunization for the subsequent quantification of IFN-γ and NO levels in serum.

### Measurement of IFN-γ levels

The quantity of IFN-γ was measured by a commercial ELISA (Mouse IF-γ Duoset ELISA Development System, Genzyme, Cambridge and OPTEIA Mouse IFN-γ Ser, Pharmingen, San Diego, USA) in accordance with the manufacturer's instructions. The results were expressed as pg/ml of IFN-γ and using a standard curve of known quantities of IFN-γ.

### Measurement of nitric oxide levels

The NO production levels are taken as an indirect measurement of the nitrite and nitrate levels in serum. The measurement was performed by a chemiluminescence assay using the Sievers NOA 280 nitric oxide detector, following the method recommended by the manufacturer (Sievers Instruments Inc. 1996)

The technique used permits measuring nitrates or nitrates + nitrites depending on the NO reduction process used. The nitrites present in the samples were reduced to NO by incubation with 350 mM of NaI in glacial acetic acid according to the following reaction:

I⁻ + NO₂⁻ + 2 H⁺ ----------------→ NO⁺ ½ I₂ + H₂O

In the measurement of nitrites and nitrates, these were reduced to NO by incubation with 50 mM of VCl₃ in 1N HCl at 90°C according to the following reaction:

2 NO₃⁻ + 3 V⁺³ + 2 H₂O ---------------- → 2 NO + 3 VO₂⁺ 4 H⁺

Reduction to NO occurs in the detector tank. The resulting NO of any of the two previous reactions is transported to the detector by a vacuum pump. In the detector, the chemiluminescence reaction is produced between the NO and ozone:

NO + O₃ ---------------- → O2 + NO₂* ---------------- → NO₂ + h●

NO₂* emission is in the red and infrared region of the light spectrum and is detected by a red-sensitive photomultiplier tube. This signal is quantified and the data obtained are collected and processed by a computer.

The administration to mice of a recombinant adenovirus (RAd) which expresses mouse IL-12, generates a cascade of responses amongst which an important increase in the serum levels of IFN-γ and NO stands out. As the IFN-γ and NO induction processes are affected by TGF-β1 levels (Schini V.B. et al., 1992), we also studied the effect of the administration of p144 on said levels. Figure 2 and Figure 3 respectively indicate the levels of IFN-γ and NO in the serum of mice which have been administered a dose of 1 x 10⁸ pfu of recombinant adenovirus for mouse IL-12 (RAd IL-12) with or without p144. Figure 2 shows that the administration of peptide p144 together with RAd IL-12 increases the levels of IFN-γ induced with respect to those reached after the administration of just RAd IL-12.

Figure 3 shows the effect of the administration of RAd IL-12 with or without p144, on NO serum levels. The joint administration of RAd IL-12 and p144 generates a greater level of NO on day 6, with respect to that generated with just the administration of RAd IL-12.

The effect of p144 on this induction model of pro-inflammatory cytokines can be explained based on the action that TGF-β1 exerts on the regulation, expression and activity of IL-12 and on the processes that this cytokine activates. In fact, it is described that TGF-β1 exerts an antagonistic action on the expression and activity of IL-12 and IFN-γ. Therefore, if p144 neutralizes TGF-β1, it eliminates the antagonistic effect of this cytokine on the expression and activity of IL-12 and IFN-γ and they consequently increase the serum levels of IFN-γ (Fig. 2). In sum, in this model TGF-β1 acts by checking both the expression of IL-12, and (in concomitant manner) the expression of TGF-β1. In consequence, inhibition of TGF-β1 by p144 has the effect of increasing IFN-γ expression.

With respect to the effect that p144 has on the increase in NO, we believe that it may also be explained based on the inhibition of TGF-β1 by p144. In fact, since TGF-β1 inhibits the expression and activation of the iNOS enzyme, responsible for NO production, it is logical to conclude that if the cytokine is eliminated, NO levels will tend to increase, as is observed in Figure 3 on day 6.

Since IFN-γ induces the expression and activity of iNOS, the results of Figures 2 and 3 are coherent, as on day 6 it is observed that the administration of p144 respectively redounds in an increase in IFN-γ and NO levels.

### EXAMPLE3

### Antibody induction

To analyse the immunomodulating effect of peptide p144 on the humoral response, female BALB/c mice (Harlan, Barcelona) were used, of between 6 and 8 weeks old. For the induction of specific antibodies, recombinant adenovirus (RAd-LacZ) inactivated by heat in a bath at 100°C for 10 minutes was inoculated.

### Groups of animals and treatments

Three mice were immunized per group, by the intraperitoneal of a mixture of 200 µl which contained 1 x 10⁸ pfu of the inactivated RAd-LacZ adenovirus, physiological serum (PS) or with 50 µg of peptide p144, all emulsified in Freund's complete adjuvant (FCA) in a 1:1 volumetric ratio as indicated in table M4. Thirty days after the first immunization, the animals were reimmunized with the same mixture, but emulsified in Freund's incomplete adjuvant (FIA). Blood samples were taken from the retroorbital plexus on days 15 and 45 to quantify the anti-adenovirus antibodies generated in each animal.

In order to study the possible induction of antigen intolerance in the mice treated with p144, on day 50 after the 1^{st} injection, the mice were inoculated intravenously with 4 x 10⁸ pfu of active RAd-LacZ in 100 µl of RPMI-1640, including a new group of mice (cont iv), which had only received the intravenous dose of active LacZ adenovirus. Blood samples were taken after 7 days in all groups. Next, the animals were sacrificed to include liver samples in OCT^{®} (Tissue-Tek^{®}, SAKURA, the Netherlands) to the subsequent evaluation of LacZ expression in the liver.

**Table 1**

| **Composition of the immunisation mixtures of the different groups with heat-inactivated adenovirus** | | | | |
|---|---|---|---|---|
| Groups | RAd-LacZ | P144 | FCA/FIA | PS |
| RAd-LacZ | 50 µl (1x10⁸ pfu) | - | 100 µl | 50 µl |
| RAd-LacZ + p144 | 50 µl (1x10⁸ pfu) | 50 µl (50 µg) | 100 µl | - |

### Quantification of anti-RAd LacZ antibodies in the serum

The detection of antibodies in serum against RAd-LacZ was performed by ELISA assays using Maxisorp^{®} flat-bottomed 96-well plates (Nunc, Roskilde, Denmark), based on the streptavidin-biotin system using 2, 2'-azino-bis-3-ethylbenzthiazoline-6-sulphonic acid (ABTS) as developer. The plates were incubated throughout the night at 4 °C with 50 µl per well of a solution of 75 µl of RAd-LacZ 10¹⁰ pfu/ml in 10 ml 0.1 M Na₂CO₃ (pH=10.5). Next, 3 washes were performed with 200 µl per well of PBST wash buffer (pH=6 saline phosphate buffer with 0.1 % Tween 20). The non-specific bonds were blocked by incubating the plates for one hour at room temperature with 400 µl per well of PBST with 1 % powdered milk (PLT). The plates were emptied and three washes were performed with PBST. 4 µl of serum were added in 100 µl of PLT, making 8 double serial solutions and the plates were incubated for 1 hour in a stove at 37°C. It was washed three times with PBST and incubated for one hour at 37°C with 50 µl per well, of a 1/1000 dilution in PBST of biotinylated mouse anti-IgG antibody obtained from a goat (Amersham). It was washed three times with PBST and 50 µl per well were added of a 1/500 solution of streptavidin-peroxidase (Amersham). After 1 hour of incubation, three washes were performed with PBST and the plate was then developed. ABTS was used as a substrate of the developing reaction, which gives green colouring in the presence of hydrogen peroxide and the peroxidase enzyme. A solution was prepared with 10 ml of 0.6% acetic acid (pH=4.6), 7.5 µl of 33% H₂O₂ (v/v) and 100 µl of 40mM ABTS. 100 µl per well were added and after one hour the plate was read at 405 nm in a Multiskan Plus MKII reader (Labsystem, Helsinki, Finland).

### In situ staining of transgen expression (X-gal stain)

The cryostat cuts (6µm) from the preparations of liver samples included in a compound for optimal cutting temperature compound (OCT) were dried at room temperature. Next, they were fixed with 0.5% glutaldehyde during 10 minutes, adding 200 µl per preparation. 3 washes were next performed in PBS, to then add 200 µl of the stain mixture: 30 mM K₃Fe(CN)₆, 30 mM K₄Fc(CN)₆, 20 ng/ml X-Gal and MgCl₂ in PBS. The preparations were incubated for 12 hours at 37 °C, after which, 3 washes were performed in PBS and, once dried, the preparations were mounted.

The use of certain recombinant virus as gene therapy tools has the drawback that they can be used few times due to the induction of antibodies to the virus. Indeed, if the virus is administered more than once, its effect will be notably (or completely) reduced due to the fact that the antibodies induced in the first administrations are capable of neutralizing the virus administered in subsequent treatments. For this reason, we decided to study the role of p144 in a process of the induction of antibodies against a recombinant virus. The basic idea behind this experiment was to study if the neutralization of TGF-β1 by p144 may or may not inhibit antibody induction, or even induce immunological tolerance to adenovirus in successive administrations. Thus, in a first experiment, we immunized mice with a heat-inactivated recombinant adenovirus for Lac Z (RAd LacZinact). This immunization was performed in the presence or absence of p144. As is shown on Figure 4, the first immunization had no quantifiable effect by ELISA on the production of anti-RAd Lac Z production.

Nevertheless, a very different effect was observed after a second immunization (fig. 5). Thus, the second immunization with RAd LacZ inactivated by the presence of p144 induced high titers of antibodies against RAd LacZ. Nevertheless, as p144 is included in the immunization mixture, the antibody titer against RAd LacZ was clearly lower than that obtained with only inactivated RAd LacZ.

After these results, it was decided to study if the mice treated with p144 may have developed a degree of tolerance to the antigens shown in the form of inactivated adenovirus. To do this, the aforementioned groups of mice were inoculated by intravenous route on day 20 (after the second immunization) with active LacZ adenovirus (RAd LacZact), in the absence of peptide p144. Seven days later, the expression of transgen was expressed *in vivo*, and both the presence of antibodies in serum and the expression of LacZ in livers were quantified. As can be observed in Figure 6, the antibody titer was approximately equalled in the two groups of mice, indicating that tolerance had not been produced and that p144 is capable of inhibiting the humoral response only when it is included in the immunization mixture with the antigen, in the present case the heat-inactivated adenovirus.

The histological analysis on day 7 of the mouse livers of Figure 6 showed that only the control group infected intravenously with RAd LacZact was positive to the LacZ stain, which suggested that in the other groups, the presence of anti-adenovirus antibodies in serum was sufficient to neutralize the administration of the RAd LacZact virus, impeding the infection in the liver and the subsequent expression of the LacZ gene (Fig. 7).

### EXAMPLE 4

This example studies the effect of the presence of peptide p144 in an immunization mixture together with a peptide (FIS), which acts as a T-helper determinant. This peptide induces a cytokine profile which favours the production of anti-bodies against different antigens.

### Induction of T-helper responses

The FIS peptide is characterized as a T-helper determinant derived from sperm whale myoglobin, amino acids (106-118). This peptide has been widely used for the induction of antibodies against haptenic peptides. We wanted to analyse the effect of peptide p144 on the induction of a cytokine profile characterized among other things by the increase in IFN-γ and IL-12. In this model the following was administered by intravenous route to groups of 3 female BALB/c mice, 4 to 8 weeks old, (Harlan, Barcelona), distributing the animals in the following treatments:
- **FIS:** mice which received by intravenous route a 1:1 emulsion of Freund's incomplete adjuvant and saline serum which contained 50 µg of FIS.
- **FIS +p144:** mice which received by intravenous route a 1:1 emulsion of Freund's incomplete adjuvant and saline serum which contained 50 µg of FIS and 50 µg of p144.

10 days after immunization, the animals were sacrificed and the popliteal, inguinal and periaortic lymph nodes were extracted. The nodes were homogenized with a syringe and they were washed three times at 4°C with washing medium (clean RPMI 1640 medium). Next, the cells were resuspended in complete medium (RPMI 1640 with 10% FBS, 2 mM of glutamine, 100 U/ml of penicillin, 100 µg/ml of streptomycin, 5x10-5 M of β-mercaptoethanol, 25 mM of Hepes and sodium pyruvate), at a concentration of 5.3 x 10⁶ cells /ml adding 150 µl in each well of a flat-bottomed 96-well plate. The different peptide concentrations (6 and 10 µM) were added in triplicate and at a volume of 100 µM per well. The cells were cultured in a stove at 37°C and with 5% CO₂ for two days. 24 hours later, 50 µl of the supernatant were collected on the 96-well plate to measure IL-2 produced in the cells, and after 48 hours, 50 µl of the supernatant were collected to measure IFN-γ. These supernatants were frozen at -20 °C until the quantification of the cytokine concentration.

### Measuring IFN-γ levels

The quantity of IFN-γ was measured using a commercial ELISA (Mouse IFN-γ Duoset ELISA Development System, Genzyme, Cambridge and OPTEIA Mouse IFN-γ Set, Pharmingen, San Diego, USA) in accordance with the manufacturer's instructions. The results were expressed as pg/ml of IFN-γ using a standard curve of known quantities of IFN-γ.

### Measuring IL-2 levels

The quantity of IL-2 in each supernatant was measured by studying the proliferation of the CTL.L. cell line, whose growth is IL-2 dependent (Lai M. ct al., 1987). This line was maintained in culture with complete medium supplemented with IL-2 at a concentration of 10 U/ml.

To perform the assay, the supernatants were cultured with 3000-5000 CTL.L. cells per well, diluted to a final volume of 100 µl. After 24 hours of culture, 0.5 µCi (25 Ci/mmol) of tritium labelled thymidine (Amersham) were added per well and the cells were collected 20 hours later on plates with a filter (Unifilter-96 GF/C^{®} , Perkin Elmer) with a harvester (Filtermate 196 Harvester, Packard). The radioactivity was quantified in a scintillation counter (Top Count, Microplate Scintillation Counter, Packard) after the addition of 25 µl of scintillation liquid (MICROSCINT^{®}, Packard, Bioscience Company) per well. The results of the counts were expressed as mU/ml of IL-2, interpolating the counts of each well in a standard curve.

The FIS peptide is a T-Helper determinant which includes residues 106 to 118 of the sequence of sperm whale myoglobin. The immunization of BALB/c mice with FIS induces the activation of IFN-γ and IL-2 producing T-lymphocytes in response to the peptide. Since TGF-β1 plays a role in the induction of immune responses, the effect of p144 on cytokine production after immunization of mice with FIS was studied in the presence and absence of p144. To do this, BALB/c mice were immunized with 50 µg of FIS alone, or with 50 µg of FIS in the presence of 50 µg of p144.

As can be observed in Figure 8, the presence of peptide p144 in the immunization mixtures reduced IL-2 and IFN-γ production compared to FIS. It is important to indicate that p144 is also probably presented by the class II MHC molecules from BALB/c mice as a DTh, since when restimulating *in vitro* against p144, production of IL-2 and also some production of IFN-γ was observed. The results suggest that the inclusion of p144 in the immunization mixture has a negative effect on the DTh helper capacity, which is probably due to the neutralization of TGF-β1 which would be necessary at the time of immune response induction.

### EXAMPLE 5

### A. Effect of peptide p144 on an immunization mixture with a DTc (AH1) and a DTh (LQV) of the tumour antigen.

A DTc is a peptide which is presented by the MHC-II on the surface of the presenting cell, and a DTc is a peptide which is presented by MHC-I on the surface of the presenting cell and in tumour cells. It is known that the joint immunization of a DTc (AH1 peptide) and a DTh (LVQ peptide), both peptides coming from the gp70 protein of the tumour antigen expressed by CT26 cells, was capable of protecting against the subcutaneous growth of 500,000 CT26 tumour cells. Since TGF-β1 is important in the immune response induction process, we wanted to study the effect of peptide p144 on the induction of the response responsible for protection against the growth of CT26 cells. As is indicated in Table 1, three groups of BALB/c mice were immunized with the following mixtures of Freund's incomplete adjuvant: (i) with AH1 LVQ, (ii) with AH1 + LVQ + p144 and (iii) just with Freund's incomplete adjuvant. It was observed that only the immunization with AH1 + LVQ managed to protect the mice and that, therefore, the incorporation of p144 in the immune mixture, had a negative effect on protection against the growth with CT26 cells.

**Table 2**

| **The administration of p144 together with AH1 + LVQ has a negative effect on protection against the growth of CT26 cells reached after immunization of BALB/c mice with AH1 + LVQ** | |
|---|---|
| **Immunization mixture** | **Protection level** |
| (AH1 +LVQ) in FIA | 3/3 |
| (AH1 + LVQ + P144) in FIA | 0/3 |
| FIA | 0/3 |

Since peptide p144 is capable of blocking TGF-β1 activity, the results obtained suggested that the cytokine plays a crucial role in the induction of an effective anti-tumour response and that its blocking at this stage has a negative effect on the induction of protective anti-tumour responses. This result is in harmony with another previous one (Fig. 8) wherein it shows how the administration of p144 together with DTh FIS blocks the activation of the Th response to FIS.

### B. Effect of peptide p144 after immunization with a DTc (AH1) in a pulmonary metastasis model.

It is speculated that the neutralization of TGF-β1, once the immune response has been induced, could have a beneficial effect on the evolution thereof. To prove this concept, the effect of administrating p144 was studied at different times and different immunization protocols, on the survival of mice in a pulmonary metastasis model induced by the administration of 5 x 10⁵ CT26 cells by intravenous route.

It is known from previous experience that immunization with only DTc AH1 produced a certain delay in the appearance of tumours after the intravenous administration of CT26 cells. For this reason, in a survival experiment, the inclusion of p144 was compared at different times after immunization with AH1. Thus, the animals in group (i) just received the administration of CT26 cells, the animal groups (ii), (iii) and (iv) were immunized on day 0 by subcutaneous route with 50 µg of AH1 in FIA (Freund's incomplete adjuvant) and they were then administered 5 x 10⁵ CT26 cells by intravenous route on day 10. Group (iii) further received 50 µg of p144 in 500 µl of PS (physiological serum) by intraperitoneal route on alternate days between days 4 and 20. Group (iv), as with group (iii), received 50 µg of p144 in 500 µl of PS by intraperitoneal route on alternate days, only between days 10 and 20. As can be observed in figure 9, immunization with AH1 (ii) only mediates a slight delay in mortality with respect to the unimmunized control group (i). In the animals immunized with AH1, treatment with p144 reinforces the survival effect, especially in group (iv), wherein p144 was administered between days 10 and 20.

### C. Effect of p144 peptides after immunization with a DTc (AH1) in a subcutaneous tumour model.

Next, the effect of p144 was studied on a tumour progression model which was less aggressive than the intravenous administration of CT26. In this new model, mice were immunized with 50 µg of AH1 on day 0 and ten days later they were administered 5 x 10⁵ CT26 cells by subcutaneous route. Furthermore, with the aim of testing the effect of blocking TGF-β1 on protection against CT26 tumour cells, another two groups of mice (groups 2 and 3) were treated on alternate days by intraperitoneal route between days 10-30 with 50 µg of p144.

**Table 3**

| **The administration of p144 after an immunization with AH1 has a positive effect on protection against the growth of CT26 cells in BALB/c mice with AH1** | | | | |
|---|---|---|---|---|
| **Groups** | **Day 0** | **Day 10** | **Day 10-30** | **Animals protected on day 50** |
| 1 | 50 µg AH1 | 5 x 10⁵ CT26 | PS | 0/10 |
| 2 | 50 µg AH1 | 5 x 10⁵ CT26 | 50 µg p144 every 48 hours | 4/10 |

As can be observed in Table 3, the blocking of TGF-β1 ten days after immunization with AH1 generates a protection against the tumour growth measured on day 50 after the subcutaneous administration of tumour cells. This protection reached 40% of the animals.

Given the increase in effectiveness of protection against the growth of tumour cells, due to the neutralization of TGF-β1, it is of great interest and can be a strategy to adopt in order to obtain better anti-tumour responses.

### EXAMPLE 6

### Modulation of NK cells

### Materials and Methods

### NK cell cultures

They were performed from spleen cells from RAG1^{-/-} mice, without T and B lymphocytes. In some cases, the total splenocytes was cultured on 6-well plates at 4x10⁶ cells per ml of RPMI medium enriched with 10% SBF, L-glutamine, antibiotics, non-essential amino acids, β-mercaptoethanol and human recombinant interleukin-2 (Chiron) at 600 IU/ml. In half the wells, peptide p17 was added at a concentration of 150 µg/ml. After 48 hours, the medium was removed and the wells were washed with RPMI medium to discard the non-adherent cells. Next, fresh medium was added with/without peptide. On day +5, the medium was again changed, this time replacing all the cells and peptide p17 in the corresponding cultures. The cells counts with trypan blue were performed on days +5 and +6 of this culture. In other cases, NK cells from mouse splenocytes were purified by an immunomagnetic selection using the MiniMACS system, anti-DX5 beads and MS columns (Miltenyi Biotech) according to the manufacturer's instructions. The cells thus obtained were cultured on 48-well plates at 1.5x10⁶/ml in the aforementioned medium with/without peptide p17 at 150 µg/ml). After 48 h, new peptide was added to the cells which had p17 and the counts were performed on days +2 and +4.

### Flow cytometry

The following PE-labelled rat anti-mouse monoclonal antibodies were used: anti-CD25, anti-CD69 and an isotope control antibody, all from Pharmingen (BD). The acquisition and analysis of the samples were performed using a FACScalibur and the CellQuest program.

### Proliferation assay with tritium labelled thymidine

For this assay, DX5+ cells were used on days 2 and 4 of culture. Briefly, triplicates of 10000 cells per well were plated with and without peptide, measuring their incorporation of tritium labelled thymidine in the typical culture medium with 6000 IU/ml IL-2, 6 hours after the addition of thymidine.

### Chrome release assay

The cytotoxicity of NK cells was verified by standard 4.5h ⁵¹Cr release assays. Briefly the targets were incubated with 50 µCi of ⁵¹Cr during 2 h, they were washed (3 times) and, next, the effector cells were added in different proportions, the maximum being 40:1 (effector: targets). Finally, the release of ⁵¹Cr due to lysis by NK cells was measured after 4.5 h in a TopCount Scintillation counter (Perkin Elmer). The cytotoxicity was measured as a percentage of Cr released with respect to the total acquired by the cells.

### Cell lines

The following tumour lines were used as targets of the cytoxicity assays by NK cells: MC38 (colon carcinoma) and LLC (lung carcinoma), originated in C57BL/6 and CT26 mice (colon carcinoma) and RENCA (renal carcinoma) from BALB/c mice. LLC and RENCA were cultured with RPMI supplemented with foetal bovine serum, antibiotics and L-glutamine and MC38 and RENCA in DMEM supplemented in the same way.

### Results

Peptide p17 exerts a clear anti-proliferative effect on the population of NK cells obtained from RAG1^{-/-} mice and cultured *in vitro* (Fig. 10), in proliferation quantification assays by direct cell count or DNA synthesis (incorporation of tritium labelled thymidine). When the effect of p17 on the expression on the cell surface of different markers was analysed, it was found that p17 reduces the levels of CD25 and CD69 (Fig. 11) measured as mean fluorescence intensity. The CD25 and CD69 markers mediate immunodepression and both are induced by TGF-β1. Thus, peptide p17 acts by blocking the effect of TGF-β1, on the induction of these markers (CD25 and CD69), in this cell population of the immune system. On the other hand, the cytotoxicity assays opposing this cell population to different mouse tumour lines (Fig. 12), the presence of peptide p17 improves the cytotoxic activity of this population of Natural Killer cells to a greater or lesser extent. In all experimental models, it is concluded that peptide p17 exerts a clear biological activity on the proliferation, differentiation and effector phase of the NK cells.

### EXAMPLE 7

### Modulation of dendritic cells

### Materials and Methods

### Obtainment of dendritic cells (DC) from mouse bone marrow:

Firstly, the legs were separated and placed on a plate with 10% RPMI FBS on ice. To obtain the bone marrow, it is necessary to cut the heads off the femurs and pass medium through the interior of the bone to drag the marrow to a dish with 10% RPMI FBS. Next, the bone marrow was broken up with the aid of a syringe and the contents were collected in a Falcon tube which was centrifuged at 2000 rpm for 5 minutes; after the centrifugation, the supernatant was removed and the erythrocytes were lysated, which was performed with the ACK lysis buffer. Once the cells were lysated, the depletion of those cell populations not of interest was performed; to do this, both commercial antibodies and antibodies produced from ascites combined with rabbit complement were used. The depletion was performed at a cell concentration of 2x10⁷ cells/ to which the following mixture was added:
- AntiCD4 asctites at 100 µg/ml.
- AntiCD8 asctites at 100 µg/ml.
- B220 supernatant in 1/20 dilution (B antilymphocytes)
- 10 µl/ml of GR1 (antigranulocytes)
- Complement in 1/20 dilution

This mixture was incubated for 50 minutes at 37°C stirring approximately every 15-20 minutes. After this time has elapsed, a wash was performed with clean RPMI medium and the number of cells obtained was quantified. Finally, the cells were seeded in 12-well plates at a final concentration of one million cells per millilitre (3ml/well) and 20 µg/ml of IL-4 and GM-CSF cytokines. On days and 3 and 5 after seeding, 2 ml was removed from each one of the cells which was replaced with fresh medium together with the cytokine concentration corresponding to that volume.

On day six the CD were collected, they were quantified and were placed on 12-well plates at a concentration of 1 million/ml (3 ml/well) and 20 µg/ml of the IL-4 and GM-CSF cytokines and the following treatment was performed:

| | |
|---|---|
| - Without stimulus | - without p17 |
| | - with p17 (150 µg/ml) |
| - LPS (10 ug/ml) | - without p17 |
| | - with p17 (150 µg/ml) |
| - Poly (I:C) (100 ug/ml) | - without p17 |
| | - with p17 (150 µg/ml) |
| - 1668 (1 uM) | - without p17 |
| | - with p17 (150 µg/ml) |
| - 3TC-CG40L | - without p17 |
| | - with p17 (150 µg/ml) |

| | |
|---|---|
| **LPS**: lipopolysaccharide **Poly** (I:C): Synthetic double-stranded RNA (polyinosinic-polycytidylic acid). **1668**: oligodeoxynucleotide (ODN). **3TC-CD40L**: cell line which produces CD40 ligand. | |

The cells were left in the presence of the different stimuli for 48 hours; after this time had elapsed they were collected and quantified with the purpose of performing the **mixed leukocyte response** assay, which consists of an allogenic reaction where non-adherent spleen cells of a mouse belonging to a determined strain are opposed to dendritic cells of another different strain of mouse. The aim of this assay is to study the presenting capacity of the CD which, as it belongs to a different strain of mouse, shows a different HLA restriction which is recognised by the lymphocytes of the other mouse making them proliferate. The degree of proliferation is determined through the incorporation of tritium labelled thymidine. This parameter indicates the effectiveness with which the CD present antigen. In this case, the non-adherent cells were obtained from a BALB/c mouse and the dendritic cells derived from a C57 mouse.

Furthermore, the cells were incubated with different stimuli to see the effect of p17 in this context.

### Results

Peptide p17 is capable of increasing lymphocyte proliferation in mixed leukocyte response assays (MLR) as a consequence of an increase in the effectiveness with which the cells present antigen. This effect of peptide p17 is produced with dendritic cells, non-stimulated or stimulated with LPS or pIC (Fig. 13). Nevertheless, other stimuli (1668 and 3T-CD40L) do not permit peptide p17 to mediate a difference in the effectiveness of the antigen presentation and, consequently, in the proliferative lymphocyte response. These results reveal the potential of a peptide inhibiting TGF-(β1 in the stimulation of antigen presenting cells (CD) and the effectiveness of the antigen presentation.

### EXAMPLE 8

### Modulation of regulatory T lymphocytes

### Materials and Methods

**1. OBTAINMENT OF TOTAL SPLENOCYTES:** To obtain splenocytes from 6-week old, female Balb-c mice, 4 animals were sacrificed, and after extracting the spleen, they were transferred to a clean medium for their dispersion with crystals, the homogenate was filtered (70-micron filter) and transferred to 50-ml tubes, for their subsequent washing and centrifugation. The cells obtained were incubated for 1 minute in lysis buffer, for the elimination of erythrocytes and they were subsequently washed with clean culture medium. Finally, the cells obtained were resuspended in 1 ml of AUTOMACS medium and they were counted.
**2. PURIFICATION OF CD25+ LYMPHOCYTES:** the purification of CD25+ lymphocytes was performed by the use of magnetic columns labelling with CD25PE and after incubation, anti-PE magnetic microspheres (Phycoerythrin) were added. After incubation, washing and filtration (30-micron filter) the samples were passed through a magnetic column, obtaining the eluate containing the CD25- population by gravity. Once the column is extracted from the magnetic field it is washed under pressure, obtaining the CD25+ cells.
**3. SUPPRESSANT ACTIVITY ASSAY:** To verify the regulating nature of the CD25+ population, a total of 100,000 splenocytes was seeded per well on a U-bottomed 96-well plate, in a volume of 200 µl per well and anti-CD3 antibody (0.5 µl/well) alone or opposing CD25+ or CD25-, placing 25,000 cells per well (CD25+) or 50,000 cells per well (CD25-), performing double dilutions of the concentration of these cells.
**4. ASSAY FOR THE INHIBITION OF SUPPRESSANT ACTION BY PEPTIDES:** On the U-bottomed 96-well plate, with 200 µl vol/well, 100,000 splenocytes were seeded per well and anti-CD3 antibody (0.5 µl/well) plus 25,000 CD25+ lymphocytes per well. Peptides were added to these mixtures (3 columns/peptide, 50 MicroM in the 1^{st} row and double dilution in the following three rows). 3 peptides were assayed: P17(1-11) SEQ ID NO: 3, P17(1-11)am SEQ ID NO: 4 and AcP17(1-11)am SEQ ID NO: 5. Both this assay and that of the suppressant activity were incubated at 37°C for 48h, tritium labelled thymidine was added at 0.5 µCi/well and it is harvested after 8h, subsequently counting the CPM emitted by the cells from each well.
**5. VERIFICATION OF THE EFFECTIVE SEPARATION OF THE Treg:** Labelling with fluorescent anti-CD4 and anti-CD25 antibodies and analysis by flow cytometry (89% of the population magnetically separated is DC4+CD25+).

### Results

THE CD25+ SELECTED ARE T LYMPHOCYTES WITH REGULATING ACTIVITY: Fig. 14 shows that the population of splenocytes proliferates in the presence of a suitable stimulus (AntiCD3) and in the absence of regulator cells. The presence of CG25+ lymphocytes produces a complete inhibition of the proliferation of the total splenocytes.

THE INHIBITORY PEPTIDES OF TGFB ARE CAPABLE OF BLOCKING THAT SUPPRESSANT ACTIVITY OF regulatory T lymphocytes (CD25+): Based on the model established in figure 14, the peptides derived from peptide p17 are capable of the dose-dependant blocking of the antiproliferative effect of the CD25+lymphocyte population. At a concentration of 50 µM, the peptide AcP17(1-11)Am and P17(1-1)am are capable of totally inhibiting the effect of the regulatory T lymphocytes (Fig. 15).

### EXAMPLE 8

### Effect on tumour growth

### Materials and Methods

GROUPS: 4 groups of 7 female balb-c mice, of 6 weeks old/ group.
AH1+FIA s.c.
AHI + FIA s.c. + p144 (50 µg i,p./dose/mouse)
AH1 + FIA s.c. + p17(1-11) (50 µg i.p./dose/mouse)
DESIGN: AH1 + FIA: day 10; Peptides: from day -6 on alternate days until day 10: Tumour model volume: Each 3 days from day 10 to day 41. Challenge on day 0 with CT26 (500000 cells/mouse s.c. in the flank).

### Results

REDUCTION IN THE MEAN TUMOUR VOLUME BY GROUPS TREATED WITH PEPTIDES: The effect of peptides p144 and p17(1-11)Am was studied in a tumour progression model. In this model, mice were immunized with 50 µg of AH1 10 days before the administration of 5 x 10⁵ CT26 cells. In order to verify the effect of blocking the TGF-β1 on the protection against CT tumour cells, another two groups of mice were treated on alternate days by intraperitoneal route between days 6 and 10 (50 µg/mouse/48h) with peptides p144 and p17(1-11)Am. These peptides are capable of generating a protection against tumour growth measured on day 42 after the subcutaneous administration of the tumour cells. This protection reached 100% of the animals in the case of p144 (Fig. 16).

Since the increase in the effectiveness of protection against the growth of tumour cells, due to the neutralization of TGF-β1, the great interest and possible development of these peptides in strategies to adopt with the aim of improving anti-tumour therapies is reiterated.

## Claims

1. An immune response modulating agent, **characterized in that** it comprises a peptide inhibiting TGF-β1 selected from: peptide p144 whose sequence corresponds to SEQ ID NO: 1, peptide p17 whose sequence corresponds to SEQ ID NO: 2, a peptide which has at least 90% homology therewith, or fragments of the above.

2. An immune response modulating agent according to claim 1, **characterized in that** it comprises a fragment of a peptide inhibiting TGF-β1 selected from: fragment p17(1-11) defined in the sequence SEQ ID NO: 3, fragment p17(1-11)am which corresponds to SEQ ID NO: 4, and fragment Acp17(1-11)am defined by sequence SEQ ID NO:5.

3. Use of a modulating agent defined in one of claims 1 or 2, in the regulation of humoral or cellular immune responses, or both.

4. Use of a modulating agent according to claim 3 as vaccination adjuvant.

5. Use of a modulating agent according to claim 3 in the preparation of a pharmaceutical composition for the treatment of pathologies selected from: pathologies related with microorganisms which induce an immunosupression mediated with TGF-β1 and cancer.

6. Use of an immune response modulating agent according to claim 5, **characterized in that** said microorganisms are selected from: *Leishmania, Trypanosoma cruzi,* human immunodeficiency virus, the flu virus, and the herpes simple virus.

7. Use of a modulating agent according to claim 5, **characterized in that** said composition is for the treatment of a cancer selected from: breast cancer, prostate cancer, colon carcinoma, pancreatic cancer, skin cancer, hepatocarcinoma, multiple myeloma and stomach cancer.

8. Use of a peptide inhibiting TGF-β1 selected from: peptide p144 whose sequence corresponds to SEQ ID NO: 1, peptide p17 whose sequence corresponds to SEQ ID NO: 2, a peptide which has at least 90% homology therewith, or fragments of the above, in the preparation of an immune response modulating agent.

9. Use of a fragment of a peptide inhibiting TGF-β1 according to claim 8, selected from: fragment p17(1-11) defined in the sequence SEQ ID NO: 3, fragment p17(1-11)am which corresponds to SEQ ID NO: 4, and fragment Acp17(1-11)am defined by sequence SEQ ID NO:5.

10. Use of a peptide inhibiting TGF-β1 according to one of claims 8 or 9, **characterized in that** said modulating agent regulates the humoral or cellular responses, or both.

11. Use of a peptide inhibiting TGF-β1 according to any one of claims 8 to 10, **characterized in that** said modulating agent has a stimulating effect on the immune response.

12. Use of a peptide inhibiting TGF-β1 according to any one of claims 8 to 11, as a vaccination adjuvant.

13. Use of a peptide inhibiting TGF-β1 according to any one of claims 8 to 10, **characterized in that** said modulating agent has an inhibiting effect on the immune response.

14. Use of a DNA sequence that encodes a peptide inhibiting TGF-β1 selected from: peptide p144 whose sequence corresponds to SEQ ID NO: 1, peptide p17 whose sequence corresponds to SEQ ID NO: 2, a peptide which has at least 90% homology therewith, or fragments of the above, to prepare an immune response modulating agent.

15. Use of a system of recombinant expression system that encodes a peptide inhibiting TGF-β1 selected from: peptide p144 whose sequence corresponds to SEQ ID NO: 1, peptide p17 whose sequence corresponds to SEQ ID NO: 2, a peptide which has at least 90% homology therewith, or fragments of the above, to prepare an immune response modulating agent.

16. Use of a DNA sequence according to claim 14 or of a recombinant expression system according to claim 15, **characterized in that** said immune response modulating agent has an effect selected from: stimulator and inhibitor of the immune response.

17. Use of a peptide inhibiting TGF-β1 whose sequence corresponds to SEQ ID NO: 1, SEQ ID NO: 2, a peptide which has at least 90% homology therewith, or fragments of one of the above in the preparation of a composition for the treatment of pathologies selected from: pathologies related to microorganisms which induce an immunosuppression mediated by TGF-β1 and cancer.

18. Use of a peptide inhibiting TGF-β1 according to claim 17, **characterized in that** said microorganisms are selected from: *Leishmania, Trypanosoma cruzi,* human immunodeficiency virus, the flu virus, and the herpes simple virus.

19. Use of a peptide inhibiting TGF-β1 according to claim 17, **characterized in that** said composition is for the treatment of a cancer selected from: breast cancer, prostate cancer, colon carcinoma, pancreatic cancer, skin cancer, hepatocarcinoma, multiple myeloma and stomach cancer.
